# EUROPEAN PATENT APPLICATION

(11) **EP 4 729 099 A1**
(43) Date of publication of application: **22.04.2026**
(21) Application number: 24207562.0
(22) Date of filing: 18.10.2024
(51) Int. Cl.: A61M 16/00

(54) **CONTROL DEVICE FOR A VENTILLATION MACHINE AND RESPIRATION DEVICE**

(71) Applicant: SALK - GEMEINNÜTZIGE SALZBURGER LANDESKLINIKEN BETRIEBSGESELLSCHAFT MBH, 5020 Salzburg (AT)
(72) Inventor: WALD, Martin, 5081 Anif (AT)
(74) Representative: Maiwald GmbH

(57) **Abstract**

A control device for controlling a ventilation machine for ventilating a patient, in particular for neonatal resuscitation, is provided. The control device (100 )comprises a mounting component (110), wherein the mounting component (110) is mechanically couplable to a respiration device (200) for fluidly coupling a patient interface (510) with one or more of an inspiratory branch (310) and an expiratory branch (320) of the ventilation machine (300), a control interface (120) configured to communicatively and/or operatively couple the control device (100) with the ventilation machine (300); and a trigger device (130) actuatable by a user (400) of the control device (100), wherein the trigger device (130) is configured to provide, in response to actuation of the trigger device (130) by the user (400), a trigger signal via the control interface (120) to the ventilation machine (300) to cause the ventilation machine (300) to initiate and/or perform a breathing stroke.

## Description

### TECHNICAL FIELD

The present disclosure generally relates to the field of respiration devices and ventilation systems. In particular, the present disclosure relates to a control device for controlling a ventilation machine for ventilating a patient, to a respiration device with such control device, and to a ventilation system with such respiration device and/or control device.

### BACKGROUND

A significant number of newborns do not start breathing on their own after birth but require respiratory support or artificial respiration (also referred to as mechanical ventilation). Typically, about 85% of newborns start breathing on their own, 10% need an additional stimulus and about 5% need respiratory support and/or mechanical ventilation. In particular, premature infants, for example newborns with a gestational age of less than 259 days, can suffer from respiratory distress and may require neonatal resuscitation.

Unlike the resuscitation of adults, where cardiac resuscitation is the primary focus, the ventilation is of utmost importance during the resuscitation of a newborn or initial care of a preterm infant. The primary cause of complications in a newborn after birth is in most cases a respiratory disorder. If adequate ventilation can be established within a short time, cardiac resuscitation is usually not required. For this reason, resuscitation in newborns always begins with ventilation and never with chest compressions as in adults. Thus, the ventilation system or equipment is the core element of a resuscitation unit for newborns.

In neonatal resuscitation, newborns who can breathe at least to a certain extent spontaneously or on its own are usually treated by continuous positive airway pressure ventilation (also referred to as CPAP treatment or ventilation). This non-invasive ventilation uses a continuous low positive airway pressure (CPAP) to keep the lungs "open" by applying a positive end-expiratory pressure (PEEP). In CPAP ventilation, an oxygen-air mixture or breathing gas is usually introduced into the child's throat with slight overpressure relative to ambient pressure. As a consequence, a residual amount of breathing gas remains in the lungs even during exhalation and prevents them from collapsing due to the positive end-expiratory pressure. Accordingly, an essential function during initial care is to establish functional residual capacity. This means that a volume of breathing gas must be introduced into the lungs, which were filled with fluid before birth, that remains in the lungs even at the end of exhalation. This is best achieved with devices that generate CPAP in the absence of spontaneous breathing. Newborns who do not or only insufficiently breathe by themselves, on the other hand, require artificial or mechanical respiration support by means of positive pressure ventilation (PPV).

For positive pressure ventilation, for example in case of respiratory arrest, resuscitator bags and/or so-called T-piece ventilation devices or systems are recommended and thus typically used in the field of neonatal resuscitation, for example using a mask as interface to the newborn or an endotracheal intubation tube (ET tube). In case of mask ventilation, newborns should preferably be ventilated with a CPAP and/or PEEP according to established guidelines. Since a CPAP and/or PEEP cannot be maintained with a resuscitator bag, T-piece devices or respiration devices have become widely used in clinical workflows for neonatal resuscitation and initial respiratory support.

Conventional T-pieces or (T-piece) respiration devices comprise three openings, one of which receives fresh breathing gas, for example supplied from a source or flow source of breathing gas (e.g. an oxygen-air mixture) of a first aid unit or resuscitator device. Another opening serves for delivering the breathing gas to the patient. This can be done either via an interface, such as a respiratory mask, which can be placed tightly over the patient's mouth and/or nose, or via a respiratory tube, an ET tube, through which the breathing gas can be fed directly into the patient's lungs. The third opening of a conventional T-piece can be designed as an outflow opening or valve to generate the PEEP and/or CPAP pressure. At rest, the entire gas can flow through this outflow opening into the environment, thereby creating the CPAP/PEEP pressure due to the outflow resistance throughout the tubing system and, accordingly, in the patient's lungs. When the patient is breathing adequately spontaneously, the inflowing fresh breathing gas is inhaled and used breathing gas is exhaled via the outflow opening, whereby the CPAP/PEEP pressure is maintained, this is the pressure does not drop to the ambient pressure, even at the end of expiration.

In the event of respiratory arrest and/or for PPV ventilation, the outflow opening on the T-piece can be occluded, for example with a finger. This causes the pressure in the entire tube system to rise rapidly, resulting in manual or mechanical inspiration and inflation of the lungs. To prevent overinflation and over-breathing and potentially causing damage to the patient's lungs, typically a pressure relief valve or expiration valve is used in neonatal resuscitator devices, which opens upon reaching a maximum pressure or release pressure, also referred to as peak inspiratory pressure (PIP). As soon as the finger releases the outflow opening on the T-piece, the pressure can drop back to the CPAP/PEEP level, resulting in exhalation.

The functionality and working principle of resuscitator devices employing T-pieces contrasts with the functionality of a ventilation machine, also referred to as ventilator, used at hospitals for mechanical ventilation and/or PPV. Such ventilation machines usually have a closed hose or tube circuit, which can be divided into two sections or branches by a respiration device, typically a so-called Y-piece. One section or branch functions as the inspiratory branch for the supply of fresh breathing gas, while the second section or branch functions as the expiratory branch for the removal of at least partially used or exhaled breathing gas. The lower or common leg of the Y-piece can be coupled to an ET tube for PPV ventilation, for example. Ventilators or ventilation machines for infants have a continuous flow of breathing gas through the inspiratory branch to the Y-piece, into the lungs and back through the expiratory branch. At the end of the expiratory branch, usually an expiration valve is arranged, which can control the pressure or CPAP pressure throughout the tube system via the machine. An inspiration occurs when the expiration valve closes, which builds up the ventilation pressure inflating the lungs. When a maximum pressure, release pressure or PIP is reached, the valve opens again and the pressure is held for a short time and then falls back to the initial pressure or CPAP/PEEP pressure, which causes an expiration. This allows for overpressure ventilation with a previously set or predetermined PIP.

Generally, the use of specialized CPAP devices for CPAP ventilation or treatment has been recommended and established standard in the initial care of newborns for years. In particular T-piece devices that function via a single-tube system, as described above, are recommended. In some cases, however, patients or newborns do not adequately adapt after birth under pure CPAP/PEEP ventilation and require mechanical ventilation. Typically, the entire ventilation equipment must be switched in those scenarios, which can be challenging in the course of routine work at hospitals and can require additional personnel. Also, double equipment may often be necessary in the initial care of premature infants, including a ventilation machine and for example a specialized CPAP device or resuscitator device with T-piece.

Moreover, all currently available T-piece ventilation devices or system are purely mechanically and/or pneumatically controlled. As a consequence, they do not have any monitoring functionality, and for example do not allow recording of events or closed-loop control of the ventilation. For instance, for pressure and volume monitoring, an external monitor must be placed between the respiration device or T-piece and the patient, but it does not communicate with the respiration device.

Also, modern ventilation machines or ventilators, on the other hand, cannot currently be used for newborn resuscitation, due to a lack of direct communication and control with the operator during initial care or resuscitation, without the operator having to divert their attention or hands from the patient to the ventilation machine.

### SUMMARY

To or overcome or at least partly mitigate the forementioned drawbacks of conventional ventilation systems, it may be desirable to provide for a control device for controlling a ventilation machine for ventilating a patient. Alternatively or additionally, it may be desirable to provide for an improved respiration device with such control device, and/or to provide for an improved ventilation system with such respiration and/or control device.

This is achieved by the subject matter of the independent claims. Exemplary embodiments are incorporated in the dependent claims and the following description.

Aspects of the present disclosure relate to a control device for controlling a ventilation machine for ventilating a patient, to a respiration device with such control device, and to a ventilation system with such respiration device. Any disclosure presented herein with reference to one aspect of the present disclosure equally applies to any other aspect of the present disclosure.

According to a first aspect of the present disclosure, there is provided a control device for controlling a ventilation machine, also referred to herein as ventilator, for ventilating a patient. The control device comprises a mounting component, which is mechanically couplable to a respiration device, wherein the respiration device is configured for fluidly coupling a patient interface with one or more of an inspiratory branch and an expiratory branch of the ventilation machine. The control device further comprises a control interface configured to communicatively and/or operatively couple the control device with the ventilation machine. The control device further comprises a trigger device actuatable and/or operable by a user of the control device, wherein the trigger device is configured to provide, in response or upon actuation of the trigger device by the user, a trigger signal via the control interface to the ventilation machine to cause the ventilation machine to perform one or more operations, for example to initiate and/or perform a breathing stroke.

Accordingly, the control device of the present disclosure can advantageously allow the user, also referred to herein as operator, to manage and/or control PPV or overpressure ventilation by controlling the ventilation machine via the trigger device of the control device. Therein, actuation and/or operation of the trigger device by the user can cause the ventilation machine to perform a breathing stroke, for example based on increasing the pressure in the inspiration branch to inflate the patient's lungs. In particular, the control device can allow for communication with and/or operational control of the ventilation machine, without the user having to divert their attention or hands from the patient to the ventilation machine, for example during initial care or resuscitation of the patient.

Also, by communicating with and/or controlling the ventilation machine, a monitoring of the ventilation, respectively, the characteristics of the ventilation can be enabled. For example, one or more parameters of the ventilation over time may be recorded and/or one or more particular events may be recorded via the ventilation machine. Moreover, closed-loop control of the ventilation machine can be enabled.

Accordingly, the present disclosure allows any ventilator or ventilation machine to be used for the initial care and/or resuscitation of newborns, such that ventilation can be monitored, electronically documented and controlled from the very first minute of life. An additional monitor is thus obsolete, which can save resources and equipment at hospitals.

It is noted that the present disclosure may be of particular advantage for neonatal resuscitation and/or for ventilating a newborn. However, the present disclosure is not limited in this respect, but can be used for ventilating a patient of any age.

Accordingly, the patient may generally refer to an individual or subject to be ventilated. In particular, the patient may refer to a newborn, a child, an infant and/or a premature infant.

The term patient interface may refer to or include any interface or means configured to establish a fluid communication or connection between the respiration device, for example a breathing gas outlet thereof, and the patient, for example a patient's throat. Exemplary patient interfaces may include a nasal prong, a pair of nasal prongs, a mask, an endotracheal tube, a laryngeal mask or any other suitable device. The patient interface may be coupled directly or by corresponding coupling means to the respiration device, for example including one or more tubes or hoses.

As used herein, the mounting component may refer to or denote a part or member of the control device configured to mechanically, chemically and/or physically couple and/or attach the control device to a respiration device. The mounting component may be configured to fixedly or permanently attach the control device to the respiration device, or to detachably attach the control device to the respiration device. The latter may particularly allow to re-use the control device with a plurality of respiration devices.

Optionally, the mounting component can be an integral part of the respiration device. For example, the mounting component may be constituted or provided by a wall, side or face of the respiration device.

The control interface may be configured to communicatively couple the control device with the ventilation machine. This may mean that information, data, signals and/or data signals can be transferred, unidirectionally or bi-directionally, between the control device and the ventilation machine via the control interface. Alternatively or additionally, the control interface may be configured to operatively couple the control device with the ventilation machine. This may mean that the control interface can be configured to transmit one or more signals or control signals to the ventilation machine to operatively control the ventilation machine, for example to execute one or more operations or functions at the ventilation machine.

It should be noted that various different types of communication between the control device, respectively, its control interface and the ventilation machine can be used in the context of the present disclosure. This includes analogue signals and digital signals being exchanged between the control device and the ventilation machine. Accordingly, the control interface can be configured to communicate with and/or control the ventilation machine based on providing one of or both analogue and digital signals

In the context of the present disclosure, the trigger signal can refer to a control signal provided by the control device to the ventilation machine to operatively control the ventilation machine, for example to execute, trigger and/or initiate one or more operations or functions at the ventilation machine, such as e.g. to initiate and/or perform a breathing stroke. Therein, the trigger signal can be a digital signal or an analogue signal.

As used herein, the trigger device may refer to a control means, which is actuatable, activatable and/or operable by the user. Therein, an actuation, activation and/or operation of the trigger device by the user may be detected by means of one or more sensors, switches or other components of the trigger device configured to detect one or more of a contact, a touch, an actuation, an activation and/or an operation of the trigger device by the user. Accordingly, actuation of the trigger device by the user may refer to or include contacting the trigger device by the user, for example by one or more fingers or the user's hand. Alternatively or additionally, the trigger device may be configured for touchless operation, activation and/or actuation. The trigger device may generally be configured to generate and/ or provide the trigger signal in response to or upon one or more of an actuation, an operation and an activation of the trigger device by the user.

In an example, the trigger device may be manually actuatable, activatable and/or operable by the user, for example using one or more fingers or a hand of the user. Alternatively or additionally, the trigger device may be configured for touchless operation, for example based on voice control, gesture control, eye control, or the like.

According to an embodiment, the trigger device includes one or more of:
- a switch actuatable by the user,
- a mechanical switch actuatable by the user,
- an electronic switch actuatable by the user,
- a pressure sensor for detecting a pressure or contact with the user,
- a touch sensor for detecting a contact with the user,
- a pressure capsule for detecting a pressure or contact with the user,
- a capacitive sensor for detecting a contact with the user,
- an inductive sensor for detecting a with the user,
- an optical sensor for optically detecting actuation of the trigger device, e.g. based on detecting a gesture and/or eye movement of the user,
- an acoustic sensor for acoustically detecting actuation of the trigger device, e.g. based a voice command or noise of the user,
- a microphone for acoustically detecting actuation of the trigger device, e.g. based a voice command or noise of the user, and
- an actuation sensor or device for detecting actuation, activation and/or operation of the trigger device by the user.
It should be noted that the trigger device can include a plurality of the forementioned switches, sensors and devices, for example allowing for both manual actuation and touchless actuation of the trigger device.

According to an embodiment, the control interface is configured for one or both wired and wireless communication with the ventilation machine. In other words, the control interface may be communicatively and/or operatively couplable to the ventilation machine by wireless connection or by wired connection.

For example, the control interface may be based on or be configured for one or more of USB (Universal Serial Bus) communication, LAN (Local Area Network) communication, Wireless LAN communication, Wireless Personal Area Network communication (WPAN), Bluetooth communication, Bluetooth Low Energy (BLE) communication, infrared (IR) communication, nearfield communication (NFC), radio communication, cellular network communication, an electronic communication, BUS communication, and pneumatic communication. The actual communication or data exchange between the control device and the ventilation machine may be based on a corresponding communication standard or protocol, such as for example the USB, IEEE 802.11 (Wi-Fi or WLAN), Bluetooth (including BLE), Zigbee, 3G, 4G, 5G, 6G, LTE, EDGE, or a proprietary communication protocol or standard.

According to an embodiment, the control interface is configured for one or more of an electric connection, and a pneumatic connection with the ventilation machine. An electric connection between the control interface and the ventilation machine may, for example, be established by one or more wires or cables interconnecting the control interface and a corresponding interface of the ventilation machine. Such electric connection may thus allow for or enable a communication (an electric or electronic communication) of the control device with the ventilation machine based on an electronic trigger signal, which may be an analogue or digital signal. A pneumatic connection may, for example, be established by one or more tubes or hoses fluidly interconnecting the control interface and a corresponding interface of the ventilation machine. Such pneumatic connection may thus allow for a communication (e.g. pneumatic communication) between the control device and the ventilation machine, for example based on a pressure or pressure change in the one or more tubes or hoses, which may constitute or form a pneumatic trigger signal (e.g. be caused or triggered by actuation of the trigger device by the user).

According to an embodiment, the trigger device includes a pressure capsule for detecting a pressure exerted by the user to actuate the trigger device, wherein the control interface is configured for a pneumatic connection and/or communication with the ventilation machine. In an example, the pressure capsule may comprise a compartment or volume filled with or comprising a fluid, for example air. Actuation of the pressure capsule by the user may lead to an increase in pressure in the pressure capsule, for example due to a decrease in volume of the compartment. Such increase in pressure may, for example, constitute the trigger signal, which in this case can also be referred to as pneumatic trigger signal, or may be used to generate the trigger signal, for example based on amplification. The pressure capsule may be fluidly coupled and/or may be in fluid communication via the control interface and one or more tubes or hoses with the ventilation machine, such that the increase in pressure and/or the trigger signal can be transmitted or provided to the ventilation machine to cause the ventilation machine to perform one or more operations, e.g. to initiate and/or perform a breathing stroke. Generally, using a pressure capsule as trigger device and/or using a pneumatic signal as trigger signal can allow for a direct communication and operative control of the control device with the ventilation machine. In particular, the trigger signal may be transmitted to the ventilation machine substantially without time delay. Also, a pressure capsule and pneumatic connection may almost not be error prone, as only few components are required, for example when compared to other connection means or types.

According to an embodiment, the control interface is communicatively and/or operatively couplable to the ventilation machine via one or more tubes or hoses to establish a fluid communication between the control interface and the ventilation machine. By means of such one or more tubes or hoses, the trigger signal can be reliably and quickly be provided to the ventilation machine, for example without substantial time delay.

According to an embodiment, the trigger device is configured to generate the trigger signal in response to the actuation by the user. Alternatively or additionally, the trigger device may be configured to transmit the trigger signal to the ventilation machine via the control interface upon or in response to actuation by the user.

Optionally, generating the trigger signal may comprise converting an actuation signal indicative of the actual actuation or contact of the trigger device by the user into the trigger signal. For instance, a pressure or force exerted by the user onto the trigger device may be converted into an electronic trigger signal that can be provided via the control interface to the ventilation machine. Other examples may include converting an optical signal received with the trigger device or corresponding optical sensor into an electronic, pneumatic or other signal. Yet other examples may include converting a pressure signal, a pneumatic signal, an inductive signal, an acoustic signal or a capacitive signal into the trigger signal. Alternatively or additionally to signal conversion, generating the trigger signal may include amplifying an actuation signal indicative of the actual actuation or contact of the trigger device by the user.

According to an embodiment, the trigger signal is indicative of one or more of an actuation of the trigger device by the user, a time instant of the actuation by the user, a duration of the actuation of the trigger device by the user, a force applied by the user for the actuation of the trigger device, a pressure applied by the user for the actuation of the trigger device, and a pattern of a plurality of actuations of the trigger device by the user. Alternatively or additionally, the trigger device may be configured to generate the trigger signal in dependence of one or more of a duration of the actuation of the trigger device by the user, a force applied by the user for the actuation of the trigger device, a pressure applied by the user for the actuation of the trigger device, and a pattern of a plurality of actuations of the trigger device by the user. Generally, this may allow to execute a plurality of different operations or functions at the ventilation machine and/or to adjust one or more parameters of an actual breathing stroke performed or initiated by the ventilation machine in response to the trigger signal. In particular, this may allow to perform one or more operations at the ventilation machine, such as e.g. one or more breathing strokes, in accordance or in proportion with the actual actuation of the trigger device. In particular, an operation, e.g. a breathing stroke, can be performed by the ventilation machine that is proportional to the actual actuation of the trigger device by the user, for example proportional to a duration of the actuation, to a pressure and/or to a force applied by the user for the actuation of the trigger device. Hence, versatile control of the ventilation machine can be enabled.

According to an embodiment, the control device and/or the mounting component of the control device is mechanically couplable to the respiration device by one or more of a screw connection, a plug connection, a clamp connection, a snap-fit connection, an adhesive connection, a magnetic connection, or a combination thereof. Any one or more of these connections or coupling means can allow for a reliable and robust mechanical coupling.

According to an embodiment, the mounting component includes a strap for mechanically attaching the control device to the respiration device. By means of the strap, the control device can be detachably attached to the respiration device. This can allow to retrofit existing respiration devices and can allow to re-use the control device with multiple respiration devices.

According to an embodiment, the control device further comprises one or more of a power supply interface for supplying electrical energy to the control device, and an energy storage for storing electrical energy and/or supplying electrical energy to the control device. The energy storage may for example include one or more batteries, accumulators, or capacitors. Accordingly, the control device may be powered or supplied with electrical energy via the power supply interface and/or via the energy storage. Therein the control device may be connected to an external power supply, such as a supply grid, via the power supply interface. Alternatively or additionally, the power supply interface may be connectable to the ventilation machine, such that the control device can be powered by receiving electrical energy from the ventilation machine. It should be noted that the power supply interface and the control interface may also be combined in a common interface or interface arrangement of the control device.

According to an embodiment, the control interface is configured to supply the control interface with electrical energy via the ventilation machine. Accordingly, the control device can be powered by receiving electrical energy from the ventilation machine via the control interface.

A second aspect of the present disclosure relates to a respiration device for ventilating a patient. The respiration device comprises a body part, wherein the body part comprises a breathing gas inlet fluidly couplable to an inspiratory branch of a ventilation machine, a breathing gas outlet fluidly couplable to a patient interface for supplying breathing gas from the breathing gas inlet to the patient, and an outflow port fluidly couplable to an expiratory branch of a ventilation machine. Optionally, the respiration device may include one or more further ports, inlets, outlets or openings. The respiration device further comprises at least one control device according to the first aspect of the present disclosure.

The respiration device may primarily serve for fluidly coupling the ventilation machine with a patient interface. Therein, the respiration device may be formed as handheld component, which may either be disposed after user or be re-usable multiple times.

The body part of the respiration device may refer to a support structure, for example a substantially solid support structure, at which the breathing gas inlet, the breathing gas outlet and the outflow port may be arranged. At least a part of one or more of the breathing gas inlet, the breathing gas outlet, and the outflow port may be formed by material of the body part. Fluid coupling between one or more of the breathing gas inlet, the breathing gas outlet, and the outflow port may be provided by material of the body part or by one or more other components coupled to the body part, such as a tube, hose or other coupling. Accordingly, the body part may comprise multiple members or parts, which may assembled, or it may be formed as single part.

Generally, the respiration device and/or the body part may be manufactured from various different materials, including metal, steel, aluminium, plastic material, resin-based material, reinforced material or others. For example, at least a part of the body part and/or the respiration device may be manufactured from plastic material in a blow-moulding or moulding process.

In the context of the present disclosure, the terms outlet and outlet port as well as inlet and inlet port may be interchangeably or synonymously used. The breathing gas inlet, the breathing gas outlet, and the outflow port may each comprise or define at least one opening, aperture or orifice for receiving and/or releasing breathing gas. The breathing gas inlet, the breathing gas outlet, and the outflow port may include or define physically separate openings. For instance, the breathing gas inlet, the breathing gas outlet, and the outflow port may be formed or arranged at different parts or portions of the body part. Further, at least a part or subset of the breathing gas inlet, the breathing gas outlet, and the outflow port may optionally be in fluid communication with one another.

According to an embodiment, the respiration device and/or the body part is formed as Y-piece, T-piece or F-piece. In other words, the body part may be substantially Y-shaped, T-shaped, or F-shaped. It should be noted, however, that also other shapes or forms are possible, such as e.g. an X-shape or other shape.

Typically, the lower leg of the T-shaped, F-shaped or Y-shaped body part may form the breathing gas outlet couplable to the patient interface. One of the upper legs of the T-shaped, F-shaped or Y-shaped body part may form the breathing gas inlet or be couplable to the inspiratory branch of the ventilation machine. The other upper leg of the T-shaped, F-shaped or Y-shaped body part may form the outflow port or be couplable to the expiratory branch of the ventilation machine.

According to an embodiment, the body part includes a tubular middle section, wherein the breathing gas inlet and/or the outflow port is laterally formed at the tubular middle section of the body part. The breathing gas inlet and the outflow port may be formed at the same or different sides or portions of the tubular middle section. Alternatively or additionally, the breathing gas inlet and/or the outflow port may be arranged at an end or end face of the tubular middle section.

Optionally, at least a part of the breathing gas inlet and/or at least a part of the outflow port may laterally protrude from the tubular middle section of the body part. The breathing gas inlet and the outflow port may protrude from the tubular middle section parallel to each other, in opposite directions or transverse to each other. Optionally, the breathing gas inlet and the outflow port may be spaced apart from each along a longitudinal axis of the tubular middle section.

According to an embodiment, the breathing gas outlet is formed at an end or end face of the tubular middle section of the body part. It should be noted, that other designs and arrangements of the breathing gas outlet, for example laterally at the tubular middle section of the body part, are encompassed by the present disclosure.

According to an embodiment, the control device and/or the mounting component thereof is mechanically and/or physically coupled and/or attached to the body part. For example, the control device and/or the mounting component thereof may be mechanically and/or physically coupled to an end or side of the body part. Therein, the control device and/or the mounting component thereof may be directly coupled, e.g. such that a face of the control device and/or the mounting component thereof is in direct contact with a portion of the body part. Alternatively, the control device and/or the mounting component thereof may be indirectly coupled to the body part, e.g. such that the control device and/or the mounting component thereof is coupled to the body part via another component or element.

According to an embodiment, the control device is removably attached to the body part. As mentioned above, this may allow to retrofit and/or re-use the respiration device.

According to an embodiment, the control device and/or the mounting component is integrally formed with the body part. In other words, the control device and/or the mounting component thereof may be formed as a single part or component with the body part. Alternatively or additionally, the control device and/or the mounting component may be formed by a portion of the body part, in particular by an end portion or a side portion of the body part.

In an exemplary configuration, the mounting component may be formed, provided and/or constituted by the body part. For example, a wall of the body part may form the mounting component. In such configuration, the trigger device and the control interface may be accessible for the user. For instance, the trigger device and/or control device may protrude from the body part. Alternatively, the trigger device and/or control device may may be integrated in a wall of the body part.

According to an embodiment, the respiration device further comprising a flow sensor fluidly coupled to the breathing gas outlet. By means of the flow sensor a flow of breathing gas into the patient's lungs and exhaled therefrom can be determined and/or monitored.

A third aspect of the present disclosure relates to a ventilation system for ventilating a patient. The ventilation system comprises a respiration device according to the second aspect of the present disclosure and/or a control device according to the first aspect of the present disclosure. Further, the ventilation system includes a ventilation machine. Therein, an inspiratory branch and an expiratory branch of the ventilation machine may be coupled to the respiration device.

Further aspects of the present disclosure may relate to use of a control device, a respiration device and/or a ventilation system, as described hereinabove and hereinbelow, for neonatal resuscitation and/or respiratory support.

These and other aspects of the present disclosure will be apparent from and elucidated with reference to the embodiments described hereinafter.

### BRIEF DECSRIPTION OF THE DRAWINGS

Exemplary embodiments will be further described with reference to the Figures, wherein:
Fig. 1 shows a control device according to an exemplary embodiment;
Figs. 2A to 2E each show a respiration device with a control device according to an exemplary embodiment; and
Fig. 3 shows a ventilation system according to an exemplary embodiment.

The Figures are schematic only and not true to scale. In principle, identical or like parts, elements and/or steps are provided with identical or like reference numerals in the Figures.

### DETAILED DESCRIPTION OF EMBODIMENTS

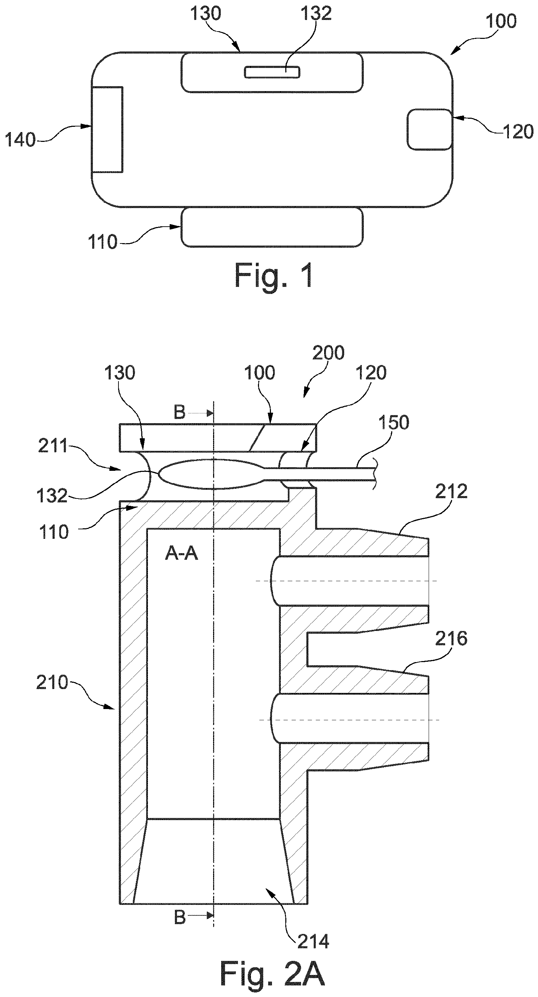

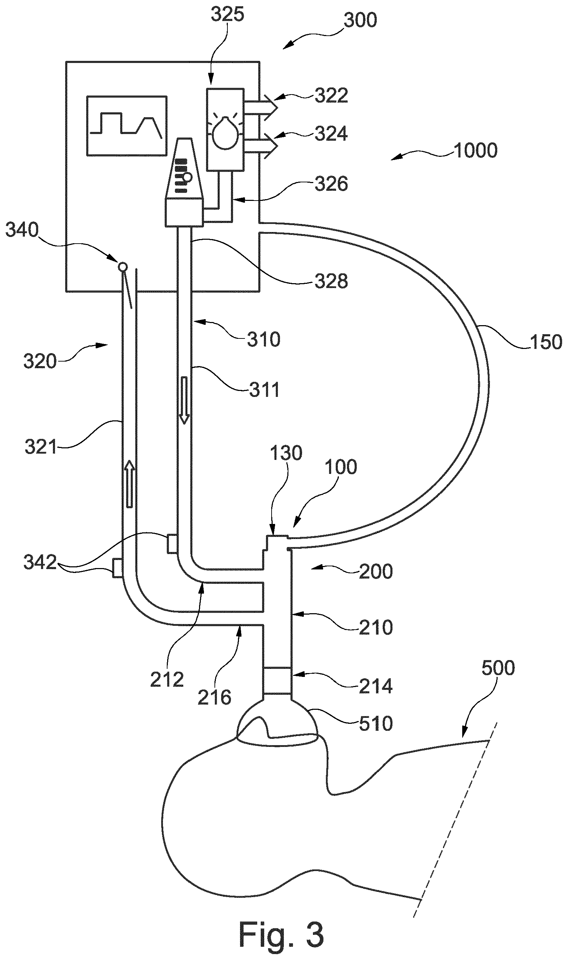

Fig. 1 shows a control device 100 according to an exemplary embodiment for controlling a ventilation machine 300 (see Fig. 3) for ventilating a patient 500 (see Fig. 3), in particular for neonatal resuscitation.

The control device 100 comprises a mounting component 110 configured to mechanically couple, fix and/or attach the control device 100 to a respiration device 200, as exemplary shown in Figs. 2A to 2E.

The mounting component 110 can be configured for fixedly attaching the control device 100 to a respiration device 200 or for detachably attaching the control device 100 to the respiration device 200. The control device 100 and/or the mounting component 110 can be mechanically couplable to the respiration device 200 by any appropriate connection means, including, for example, one or more of a screw connection, a plug connection, a clamp connection, a snap-fit connection, an adhesive connection, a magnetic connection, or a combination thereof.

By way of example, the mounting component 110 and a part of the respiration device 200 may be correspondingly or complementary formed, such that the mounting component 110 can engage with and/or can be detachably attached to the respiration device 200. Alternatively or additionally, the mounting component 110 can, for example, include a strap to attach the control device 100 to the respiration device 200.

The control device 100 further comprises a control interface 120 configured to communicatively and/or operatively couple the control device 100 with the ventilation machine 300. The control interface 120 can be configured for wired or wireless communication with the ventilation machine 300.

Exemplary wired connections can be based on an electric connection, for example using one or more wires or cables to interconnect the control interface 120 and the ventilation machine 300. Other exemplary wired connections can be based on a pneumatic connection, for example using one or more tubes or hoses to interconnect the control interface 120 and the ventilation machine 300.

Wireless communication or connection between the control device 100 and the ventilation machine 300 can be implemented based on one or more known or proprietary communication standards or protocols, such as e.g. WiFi communication, cellular network communication, Bluetooth communication, Nearfield communication, or the like.

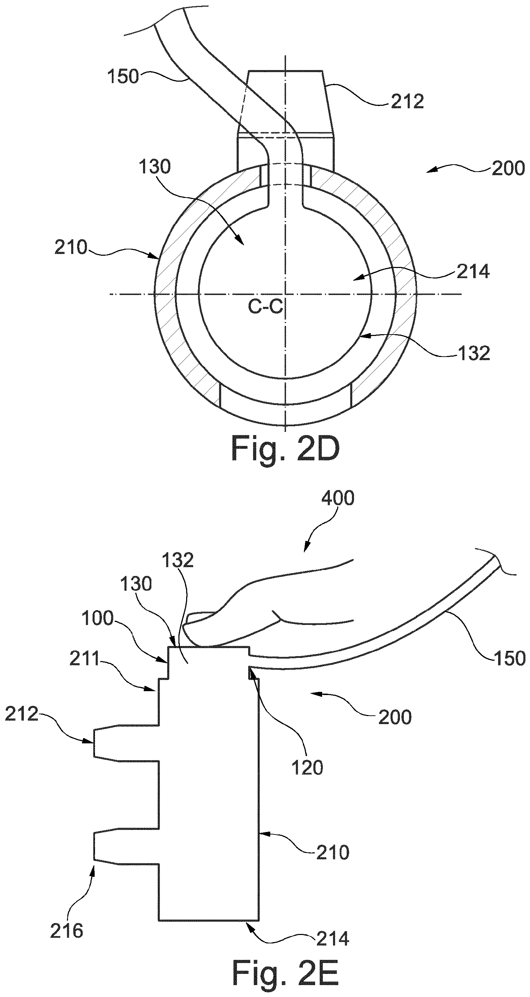

The control device 100 further comprises a trigger device 130 actuatable by a user of the control device 100. Therein, the trigger device 130 may be actuatable based on a contact of the user with the trigger device 130, for example using a finger 400 of the user of the control device (see Fig. 2E). Alternatively, the trigger device 130 may be configured for touchless actuation by the user.

For actually sensing or detecting an actuation of the trigger device 130 by the user, the trigger device 130 can include one or more sensors 132, switches 132 or other devices 132 for detecting an actuation. For example, the trigger device 130 can include one or more of a mechanical switch 132 actuatable by the user, an electronic switch 132 actuatable by the user, a pressure sensor 132 for detecting a pressure or contact with the user, a touch sensor 132 for detecting a contact with the user, a pressure capsule 132 for detecting a pressure or contact with the user, a capacitive sensor 132 for detecting a contact with the user, an inductive sensor 132 for detecting a with the user, an optical sensor 132 for optically detecting actuation of the trigger device 130, e.g. based on detecting a gesture and/or eye movement of the user, an acoustic sensor 132 for acoustically detecting actuation of the trigger device, e.g. based a voice command or noise of the user, a microphone 132 for acoustically detecting actuation of the trigger device 130, e.g. based a voice command or noise of the user, and an actuation sensor 132 or device for detecting actuation, activation and/or operation of the trigger device 130 by the user.

The trigger device 130 is configured to provide and/or generate, in response to actuation of the trigger device 130 by the user, a trigger signal via the control interface 120 to the ventilation machine 300 to cause the ventilation machine 300 to perform one or more operations, such as e.g. to initiate and/or perform a breathing stroke. Generally, the trigger signal may relate to a control signal indicative or informative of an actuation of the trigger device 130 by the user. Depending on the type of the trigger device 130, respectively, the type of the one or more sensors 132, switches 132 or devices 132 used for detecting an actuation, the trigger signal may be an electric signal, an analogue signal, a pneumatic signal, an acoustic signal, a digital signal, a pressure signal or any other signal informative or indicative of the actuation of the trigger device 130 by the user.

Accordingly, the trigger signal can be indicative and/or informative of an actuation of the trigger device 130 by the user. Optionally, the trigger signal can be indicative and/or informative of one or more of a time instant of the actuation by the user, a duration of the actuation of the trigger device by the user, a force applied by the user for the actuation of the trigger device, a pressure applied by the user for the actuation of the trigger device, and a pattern of a plurality of actuations of the trigger device by the user. Accordingly, the trigger device 130 can be configured to generate the trigger signal in dependence of one or more of a duration of the actuation of the trigger device by the user, a force applied by the user for the actuation of the trigger device, a pressure applied by the user for the actuation of the trigger device 130, and a pattern of a plurality of actuations of the trigger device 130 by the user. This can allow to perform one or more operations at the ventilation machine 300 based on one or more of a time instant of the actuation by the user, a duration of the actuation of the trigger device by the user, a force applied by the user for the actuation of the trigger device, a pressure applied by the user for the actuation of the trigger device 130, and a pattern of a plurality of actuations. In particular, an operation, e.g. a breathing stroke, can be performed by the ventilation machine 300 that is proportional to the actual actuation of the trigger device 130 by the user, for example proportional to a duration of the actuation, to a pressure and/or to a force applied by the user for the actuation of the trigger device 130.

Optionally, the control device 100 may include one or more of a power supply interface 140 for supplying electrical energy to the control device 100, and an energy storage 140 for storing electrical energy and/or supplying electrical energy to the control device 100.

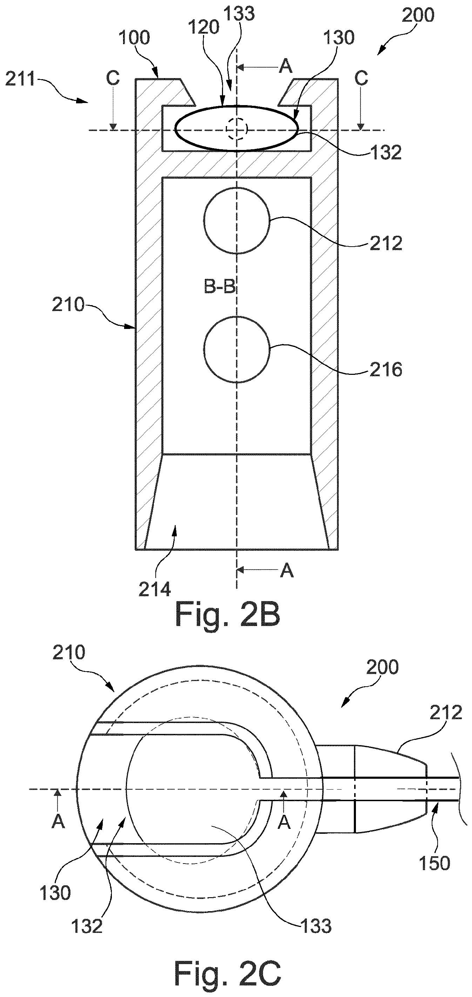

Figs. 2A to 2E each show a respiration device 200 comprising a control device 100 according to an exemplary embodiment. Therein, Fig. 2A shows a cross-sectional view along plane A-A, Fig. 2B shows a cross-sectional view along plane B-B, Fig. 2C shows a top view of the respiration device 200 with control device 100, Fig. 2D shows a top view along plane C-C, and Fig. 2E shows a perspective view of the respiration device 200 with control device 100.

Fig. 3 shows a ventilation system 1000 with a ventilation machine 300, and a respiration device 200 with a control device 100 according to an exemplary embodiment. The respiration device 200 with the control device 100 shown in Fig. 3 can for example be the respiration device 200 with control device 100 shown in Figs. 2A to 2E. Therefore, it is jointly referred to Figs 2A to 3 in the following. Also, the control device 100 shown in any of Figs. 2A to 3 comprises the same elements, components and functions as the control device 100 described with reference to Fig. 1, unless explicitly stated otherwise.

The respiration device 200 illustrated in Figs. 2A to 3 serves for ventilating a patient 500. The respiration device 200 comprises a body part 210, which comprises a breathing gas inlet 212 fluidly couplable to the inspiratory branch 310 of the ventilation machine 300, a breathing gas outlet 214 fluidly couplable to a patient interface 510 for supplying breathing gas from the breathing gas inlet 212 to the patient, and an outflow port 216 fluidly couplable to the expiratory branch of the ventilation machine 300.

In Figs. 2A to 3, the body part 210 is substantially F-shaped. The breathing gas inlet 212 is formed as one horizontal port of the F-shaped body part 210, and the outflow port 216 is formed as a second horizontal port of the F-shaped body part 210. Such F-shape, however, is exemplary only, and the body part 210 can have any other shape, including a Y-shape, and X-shape or other shape.

As shown in Fig. 3, the breathing gas inlet 212 can be coupled to a gas supply 325 or flow source 325 of the ventilation machine 300, wherein the flow source 325 can include an oxygen connection 322 for receiving oxygen, a pressure connection 324 for receiving pressurized air and an oxygen-air mixer 326 for mixing oxygen and pressurized air to provide a flow of breathing gas or fresh breathing gas into or through the inspiratory branch 310 of the ventilation machine. The inspiratory branch 310 can be coupled via one or more tubes 311 to the breathing gas inlet 212 of the respiration device 200. For instance, an outlet 328 of the mixer 326 may be coupled to the breathing gas inlet 212 of the respiration device 200 via a hose or tube 311. The breathing gas flows into the respiration device 200 via the breathing gas inlet 212 and can be delivered to the patient 500 via the breathing gas outlet 214, which may optionally be coupled via a hose or tube to the patient interface 510, for example a mask 510, an endotracheal tube 510 or other patient interface 510.

Excessive breathing gas or breathing gas exhaled by the patient 500 can further leave the respiration device 200 via the outflow port 216 that is coupled the expiratory branch 320 of the ventilation machine 300. For instance, the outflow port 216 can be coupled via a tube 321 to an expiration valve 340 of the ventilation machine 300. Based on the expiration valve 340, for example a CPAP pressure throughout the tubing system can be controlled via the ventilation machine 300.

Optionally, one or more flow sensors 342 may monitor the flow of fresh breathing gas into the breathing gas inlet 212 and/or the flow of breathing gas released through the outflow port 216. Further optionally, a flow sensor may be arranged between the breathing gas outlet 214 and the patient interface 510 to measure a flow of breathing gas into the patient's lungs and out of the patient's lungs.

The respiration device 200 shown in Figs 2A to 3 comprises a control device 100, which is integrally formed with the body part 210 of the respiration device. Accordingly, the mounting component 110 of the control device 100 shown in Figs. 2A to 3 is formed by a part or material of the body part 210.

In particular, the control device 100 is arranged at an end 211 of the body part 210, which end 211 is arranged opposite to an end of the body part 210, where the breathing gas outlet 214 is formed.

The control device 100 of Figs. 2A to 3 comprises a trigger device 130 with a pressure capsule 132 for detecting actuation of the trigger device 130 by the user's finger, as illustrated in Fig. 2E. The pressure capsule 132 and/or trigger device 130 includes an opening 133 on the top side of the respiration device 200, which can be occluded by the user to actuate or activate the trigger device 130. Therein, the opening 133 can be open or closed, for example by a membrane or the like. Accordingly, the opening 133 and/or the pressure capsule can serve as or form the trigger device 130 in the example of Figs. 2A to 3, which is actuatable by a user 400.

The opening 133 may cover at least 25%, preferably at least 40%, more preferably at least 50% of the top side or top surface of the respiration device 200, such that the user 400 can easily contact, compress, and/or actuate the pressure capsule 132 with their finger. Optionally, a wall around or defining the opening 133 may be tilted or skew towards a center of the respiration device 200 and/or the control device 100, which may further simplify handling of the device 200. The opening 133 may be circularly or half-circularly arranged or shaped towards the opening or port 120. Optionally, the opening 133 may be entirely open in opposite direction, e.g. thereby resembling the outer perimeter of the finger.

Accordingly, the control interface 120 of the control device 100 shown in Figs. 2A to 3 is configured for a pneumatic connection or communication with the ventilation machine 300. Specifically, the control interface 120 is formed as opening or port 120 that is coupled to a respective interface or port of the ventilation machine 300 by means of at least one tube 150. The at least one tube 150 may interconnect the pressure capsule 132 and a corresponding interface of the ventilation machine 300. Hence, the trigger device 130 and/or control device 100 is in fluid communication with the ventilation machine 300 via the control interface 120 and the tube 150. Opposite to the port 120, the respiration device 200 and/or control device 100 may have another opening, which may allow to insert the pressure capsule 132 into the control device 100, for example such that the at least one tube 150 extends through opening 120 or interface 120.

During initial care and/or resuscitation, the respiration device 200 is held by the user and allows for controlling or operating the ventilation machine 300, in particular to perform one or more breathing strokes. Therein, the user can actuate or activate the trigger device 130 by touching or occluding the opening 133 with the finger as shown in Fig. 2E. Upon actuation of the trigger device 130, the pressure in the pressure capsule 132 increases, and this pressure increase is transmitted as trigger signal via the tube 150 to the ventilation machine 300.

Due to the fluid communication between the ventilation machine 300 and the control device 100, the ventilation machine 300 can measure or determine the pressure, respectively the pressure increase, in the trigger device 130, pressure capsule 132 and/or tube 150. In response to detecting a pressure increase, for example above a predetermined threshold value, the ventilation machine 300 can perform a breathing stroke. Optionally, the ventilation machine 300 may be configured to determine the actual pressure exerted by the user onto the trigger device 130 and perform a breathing stroke proportional to a duration of the actuation, to a pressure of the actuation and/or to a force applied by the user to actuate the trigger device 130.

Accordingly, the ventilation machine 300 can advantageously be controlled or operated by actuating the trigger device 130 of the control device 100 with a finger. Hence, the user can obtain direct control over the ventilation machine 300 without having to divert their hands from the patient 500 or respiration device 200. Hence, the control device 100 enables the user to manage the overpressure ventilation, for example, using their finger, wherein activation of the control device 100 triggers a mechanical breath, which the ventilation machine 300 performs. Each breath can either be simply triggered or carried out proportionally to the activation of the control device 100. This means that either each activation of the control device 100 or trigger device 130 can be evaluated equally, triggering a fixed ventilation cycle or breathing stroke, or the ventilation pressure can be carried out proportionally to the strength and/or duration of the activation of the control device 100. Optionally, the ventilation machine 300 can be programmed according to the requirements of the control device 100 and can offer the user a dedicated initial care/resuscitation program.

Accordingly, by means of the control device 100 and/or the respiration device 200 including the control device 100, conventional ventilators or ventilation machines 300 can be used for the initial care and/or resuscitation of patients 500, in particular newborns. This allows the ventilation to be monitored and controlled from the very first minute of life. No additional monitor may be required for this. Moreover, it enables electronic documentation of initial care and/or resuscitation.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the claims.

As used herein, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

As used herein, the phrase "being indicative of" may mean "reflecting" and/or "comprising". Accordingly, an entity/element referred to herein as "being indicative of [...]" can be synonymously or interchangeably used herein with one or both said entity/element "comprising [...]" and said entity/element "reflecting [...]".

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

## Claims

1. A control device (100) for controlling a ventilation machine (300) for ventilating a patient (500), in particular for neonatal resuscitation, the control device (100) comprising:
a mounting component (110), wherein the mounting component (110) is mechanically couplable to a respiration device (200) for fluidly coupling a patient interface (510) with one or more of an inspiratory branch (310) and an expiratory branch (320) of the ventilation machine (300);
a control interface (120) configured to communicatively and/or operatively couple the control device (100) with the ventilation machine (300); and
a trigger device (130) actuatable by a user (400) of the control device (100),
wherein the trigger device (130) is configured to provide, in response to actuation of the trigger device (130) by the user (400), a trigger signal via the control interface (120) to the ventilation machine (300) to cause the ventilation machine (300) to initiate and/or perform a breathing stroke.

2. The control device (100) according to the preceding claim, wherein the trigger device (130) includes one or more of a switch, a mechanical switch, an electronic switch, an acoustic sensor, a microphone, a touch sensor, a pressure sensor, a pressure capsule, a capacitive sensor, an inductive sensor, an optical sensor (132), and an actuation sensor for detecting actuation of the trigger device (130) by the user (400).

3. The control device (100) according to any one of the preceding claims, wherein the control interface (120) is configured for wired or wireless communication with the ventilation machine (300).

4. The control device (100) according to any one of the preceding claims, wherein the control interface (120) is configured for one or more of an electric connection, and a pneumatic connection with the ventilation machine (300).

5. The control device (100) according to any one of the preceding claims, wherein the trigger device (130) includes a pressure capsule; and
wherein the control interface (120) is configured for a pneumatic connection and/or communication with the ventilation machine (300).

6. The control device (100) according to the previous claim, wherein the control interface (120) is communicatively and/or operatively couplable to the ventilation machine (300) via a tube (150) in fluid communication with the ventilation machine (300).

7. The control device (100) according to any one of the preceding claims, wherein the trigger device (130) is configured to generate the trigger signal in response to the actuation by the user (400); and/or
wherein the trigger device (130) is configured to transmit the trigger signal to the ventilation machine (300) via the control interface (120).

8. The control device (100) according to any one of the preceding claims, wherein the trigger signal is indicative of one or more of an actuation of the trigger device (130) by the user (400), a time instant of the actuation by the user (400), a duration of the actuation of the trigger device (130) by the user (400), a force applied by the user (400) for the actuation of the trigger device (130), a pressure applied by the user (400) for the actuation of the trigger device (130), and a pattern of a plurality of actuations of the trigger device (130) by the user (400).

9. The control device (100) according to any one of the preceding claims, wherein the trigger device (130) is configured to generate the trigger signal in dependence of one or more of a duration of the actuation of the trigger device (130) by the user (400), a force applied by the user (400) for the actuation of the trigger device (130), a pressure applied by the user (400) for the actuation of the trigger device (130), and a pattern of a plurality of actuations of the trigger device (130) by the user (400)

10. The control device (100) according to any one of the preceding claims, wherein the control device (100) and/or the mounting component (110) of the control device (100) is mechanically couplable to the respiration device (200) by one or more of a screw connection, a plug connection, a clamp connection, a snap-fit connection, an adhesive connection, a magnetic connection, or a combination thereof.

11. The control device (100) according to any one of the preceding claims, wherein the mounting component (110) includes a strap for mechanically attaching the control device (100) to the respiration device (200).

12. The control device (100) according to any one of the preceding claims, further comprising one or more of:
a power supply interface (140) for supplying electrical energy to the control device (100); and
an energy storage for storing electrical energy and/or supplying electrical energy to the control device (100).

13. The control device (100) according to any one of the preceding claims, wherein the control interface (120) is configured to supply the control interface (120) with electrical energy via the ventilation machine (300).

14. A respiration device (200) for ventilating a patient (500), the respiration device (200) comprising:
a body part (210), wherein the body part (210) comprises a breathing gas inlet (212) fluidly couplable to an inspiratory branch (310) of a ventilation machine (300), a breathing gas outlet (214) fluidly couplable to a patient interface (510) for supplying breathing gas from the breathing gas inlet (212) to the patient (500), and an outflow port (216) fluidly couplable to an expiratory branch (320) of a ventilation machine (300); and
at least one control device (100) according to any one of the preceding claims.

15. The respiration device (200) according to the preceding claim, wherein the respiration device (200) and/or the body part (210) is formed as Y-piece, T-piece or F-piece.

16. The respiration device (200) according to any one of claims 14 and 15, wherein the control device (100) is mechanically coupled to the body part (210), in particular to an end (211) or a side of the body part (210).

17. The respiration device (200) according to any one of claims 14 to 16, wherein the control device (100) is removably attached to the body part (210).

18. The respiration device (200) according to any one of claims 15 to 17, wherein the control device (100) is integrally formed with the body part (210); and/or
wherein the control device (100) is formed by a portion of the body part (210), in particular by an end portion of the body part (210)

19. The respiration device (200) according to any one of claims 15 to 18, further comprising a flow sensor (342) fluidly coupled to the breathing gas outlet (214).

20. A ventilation system (1000) for ventilating a patient (500), the ventilation system (1000) comprising:
a respiration device (200) according to any one of claims 15 to 19; and
a ventilation machine (300).
